# EUROPEAN PATENT SPECIFICATION

(11) **EP 3 970 638 B1**
(45) Date of publication and mention of the grant of the patent: **29.07.2026**
(21) Application number: 20934887.9
(22) Date of filing: 26.12.2020
(51) Int. Cl.: A61B 17/16

(54) **HOLLOW DRILL FOR MEDICAL USE**
HOHLBOHRER FÜR MEDIZINISCHE ZWECKE
FORET CREUX À USAGE MÉDICAL

(30) Priority: 14.05.2020 JP 2020085351
(43) Date of publication of application: 23.03.2022
(73) Proprietor: Bic Tool Co., Ltd., Tottori 689-3553 (JP)
(72) Inventor: ARAI, Koichi, Saihaku-gun, Tottori 689-3553 (JP); ARAI, Giichi, Saihaku-gun, Tottori 689-3553 (JP); KIMURA, Katsuyo, Saihaku-gun, Tottori 689-3553 (JP); TESHIMA, Satoshi, Saihaku-gun, Tottori 689-3553 (JP)
(74) Representative: Isern Patentes y Marcas S.L.
(86) International application number: PCT/JP2020/049048
(87) International publication number: WO 2021/229854

(56) References cited:
- EP-A1- 2 849 656
- EP-B1- 2 849 656
- WO-A1-2014/026871
- CN-U- 204 260 785
- CN-U- 210 447 179
- JP-A- 2016 155 178
- JP-A- 2018 108 223
- JP-A- 2018 108 223
- JP-A- 2019 536 507
- US-A- 5 173 014
- US-A- 5 429 504
- US-A1- 2019 125 408

## Description

### [FIELD OF THE INVENTION]

The present invention relates to a hollow drill bit for medical use.

### [BACKGROUND OF THE INVENTION]

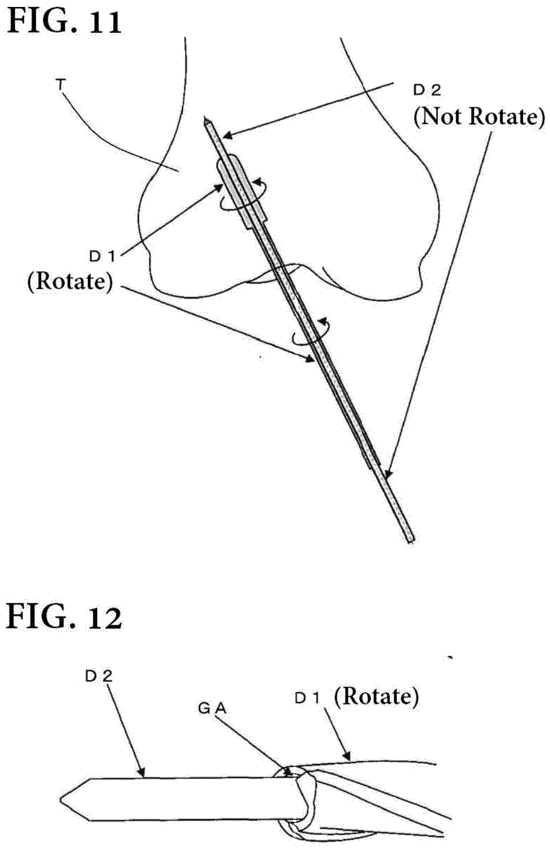

A hollow drill bit for medical use (hereinafter, simply referred to as "hollow drill bit") is a drill bit used mainly by orthopedic surgeons for reconstruction of bones, joints, ligaments or the like within the human body, and is directed to drilling holes in bones (See FIGs. 11 to 14). This drill bit is used in such a method that first, the elongated rod-like long drill bit (hereinafter, simply referred to as "small-diameter drill bit") with the drill-like cutting edges arranged at the tip end, which is called guide pin or guide wire and which is slightly thinner than the hollow portion, drills a hole in the subject bone or joint or the like, and then is fixed thereon, and is inserted in the center (hollow portion) of the hollow drill bit, and using this as a guide, drills the hole by rotation such that the hole is expanded to the intended diameter (external diameter of the hollow drill bit).

In a hollow drill bit for medical use, the present applicant has proposed a drill bit for medical use with a characterized tip end shape in order to enhance the cutting ability of the tip end of the hollow drill bit; the problem to be solved by which being to provide a drill bit for medical use that is resilient to abrasion and excellent in cutting ability by defining the shape for each cutting edge, and based on the shape, creating and arranging two or more cutting edges on the circumference of the drill bit, from the inner end of the tip end to the outer periphery (See Patent Document 1). However, the medical industry has pointed out that when the hollow drill bit (D1) is fixed with the small-diameter drill bit (D2) at the center of rotation of the hollow drill bit (D1) and then the hollow drill (D 1) is rotated to drill a hole in the bone (T), bone tissue, which corresponds to cutting chips, may not be completely discharged to the outside of the hollow drill bit (D1) and may enter the subtle gap between the small-diameter drill bit (D2) and the hollow drill bit (D1) (See FIGs. 11 to 14).

If the bone tissue enters this gap (GA), following issues may occur:
- the hollow drill bit (D1) is impeded from rotating.
- the fixed small-diameter drill bit (D2) starts co-rotating, and thus starts drilling unexpectedly.
- if the small-diameter drill bit (D2) inadvertently continues drilling the bone (T), the small-diameter drill bit (D2) may drill through the bone (T) and cause unexpected accidents such as damage to surrounding nerve.

The operating physician must pay careful attention upon drilling the bone such that the small-diameter drill bit (D2) fitted into the hollow portion (H) (See FIG. 13) of the hollow drill bit (D1) will not rotate. There are said to be some cases where the physician performs an operation while holding the small-diameter drill bit (D2) with his/her hand to prevent it from rotating.

The present inventors have designed a cutting edge shape that allows the bone tissue to be discharged to the outside of the hollow drill bit as chips while ensuring a gap like a conventional drill bit since when the small-diameter drill bit is fixed and the hollow drill bit is rotated, the hollow drill bit does not rotate smoothly unless there is an appropriate gap therebetween, and the present inventors have earnestly considered to solve the problems.

The present inventors have studied the conventional drill bit shape, and as a result, they have found that due to the structure in which the inner peripheral hollow portion of the cutting edges at the tip end is used at the beginning of drilling process, if the cutting edges at the tip end have poor cutting ability, chips may not be discharged to the side of the outer periphery and may enter the gap between the hollow portion and the small-diameter drill bit (FIGs. 12 to 13).

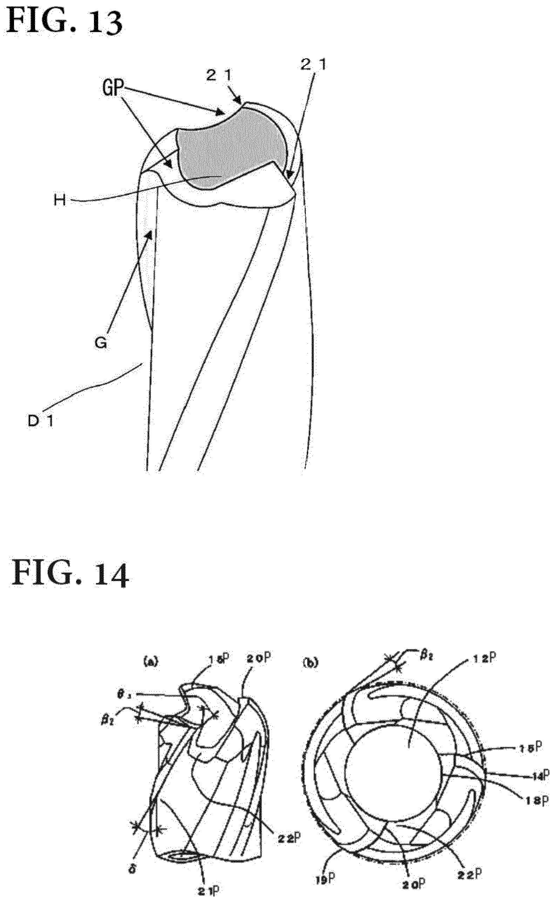

With respect to the invention described in Patent Document 1 (JP 2018 108223 A) (See FIG. 14). where (a) of FIG. 14 is a view when viewed from a slightly left side of the front of the tip end, and (b) is a top view thereof. In (a) of FIG. 14, each cutting edge (15P) has a relief angle (β₂) on the back surface. The relief angle (β₂) is set to satisfy 1°<β₂<20°. The tip end (13P) is provided with chip discharging grooves (21P) from the inner periphery end (18P) to the outer periphery (19P). The chip discharging grooves (21P) have helixes in the axial direction. The angle of the helix (helix angle (β)) of each of the chip discharging grooves (21P) is set to satisfy 5°<δ<45°. The chip discharging grooves (21P) are connected to the cylinder-shaped portion (16P) so that chips are discharged to the outer periphery (19P) at the time of drilling. Each cutting edge (15P) has a relief angle (β₂) on the back surface and chip discharging grooves (21P) have helixes so that a rake angle (θ₃) is formed on the forward surface of each cutting edge. The rake angle (θ₃) on the forward surface of each cutting edge is set to satisfy 2°<θ₃<20°. Gash pockets (22P) are provided at the side of the inner periphery end (18P) of the cutting edges (15P). The gash pockets (22P) are provided to provide clear rakes and facilitate the discharge of chips. In short, the invention of Patent Document 1 has a structure in which the inner peripheral hollow portion of the cutting edges at the tip end is used at the beginning of drilling process, and thus cannot solve these problems mentioned earlier.

### [PRIOR ART DOCUMENT]

### [Patent Document]

[Patent Document 1] Japanese Unexamined Patent Application Publication No. 2018-108223

### [SUMMARY OF THE INVENTION]

### [Problems to be Solved by the Invention]

For this reason, the object of the present invention is to design a novel tip end shape that performs most of the drilling process so as to prevent chips from entering the gap between the hollow drill bit and the small-diameter drill bit fitted into the hollow portion of the hollow drill bit as well as to provide a structure of the outer periphery so as to allow chips to be discharged toward the outer periphery and prevent them from entering the gap portion.

### [Means for Solving the Problem]

The invention is defined in claimed 1, preferred features of the invention are defined in the dependent claims.

### [BRIEF DESCRIPTION OF DRAWINGS]

[FIG. 1] FIG. 1 is a front view of the tip end of the hollow drill bit for medical use according to Embodiment 1 of the present invention.
[FIG. 2] FIG. 2 is a bottom view of the drill bit according to Embodiment 1 of FIG. 1.
[FIG. 2-1] FIG. 2-1 is a bottom view of the drill bit according to Embodiment 1 of FIG. 1, and is an explanatory view of the tip end of the drill bit.
[FIG. 3] FIG. 3 is a right side view of the drill bit according to Embodiment 1 of FIG. 1.
[FIG. 3-1] FIG. 2-1 is a right side view of the drill bit according to Embodiment 1 of FIG. 1, and is an explanatory view of the tip end of the drill bit.
[FIG. 4] FIG. 4 is a top view of the drill bit of Embodiment 1 of FIG. 1 when viewed from a direction perpendicular to and oblique to the main cutting edges.
[FIG. 5] FIG. 5 is a perspective view of the drill bit according to Embodiment 1 of FIG. 1.
[FIG. 6] FIG. 6 is a perspective view of a different section of Embodiment 1 from the perspective view of FIG. 5.
[FIG. 7] FIG. 7 is a perspective view of the entirety of the drill bit of Embodiment 1 of FIG. 1, from the tip end to the distal end.
[FIG. 7-1] FIG. 7-1 is an explanatory view of an additional embodiment in which a small-diameter drill bit is fitted into the hollow portion of the drill bit of FIG. 1.
[FIG. 8] FIG. 8 is a side view of an example of a hollow drill bit for medical use according to another Embodiment 2 of the present invention.
[FIG. 9] FIG. 9 is a perspective view of the drill bit according to Embodiment 2 of FIG. 8.
[FIG. 10] FIG. 10 is a perspective view of a different section of the drill bit from the perspective view of FIG. 9.
[FIG. 11] FIG. 11 is an explanatory view illustrating the scene in which a hole is being made in a bone using a composite drill bit of a combination of a hollow drill bit of a conventional art and a small-diameter drill bit fitted in the hollow portion thereof.
[FIG. 12] FIG. 12 is a partial enlarged view illustrating the tip end of the conventional composite drill bit of FIG. 11.
[FIG. 13] FIG. 13 is an explanatory view illustrating the tip end of the conventional hollow drill bit.
[FIG. 14] FIG. 14 is a view illustrating a hollow drill bit according to Patent Document 1, where (a) is a view viewed from a slightly left side of the front of the tip end, and (b) is a top view thereof.
[FIG. 15] FIG. 15 is a front view of the tip end of the hollow drill bit for medical use according to Embodiment 4 of the present invention.
[FIG. 16] FIG. 16 is a bottom view of the drill bit of Embodiment 4 of FIG. 15.
[FIG. 17] FIG. 17 is a perspective view of the tip end of the hollow drill bit for medical use according to Embodiment 4 when viewed from direction A of FIG. 15.
[FIG. 18] FIG. 18 is a perspective view of the tip end of the hollow drill bit for medical use bit according to Embodiment 4 when viewed from direction B of FIG. 15.
[FIG. 19] FIG. 19 is a reference view of the hollow drill bit for medical use according to Embodiment 4 when viewed from direction B of FIG. 15, and shows a structure on the assumption that a deep hole will be made.
[FIG. 20] FIG. 20 is a bottom view of the tip end of the hollow drill bit for medical use according to Embodiment 4 when viewed from direction B of FIG. 15.

### [DETAILED DESCRIPTION OF THE INVENTION]

Embodiments of a hollow drill bit for medical use are described in detailed below with reference to the accompanying drawings.

### [Embodiment 1]

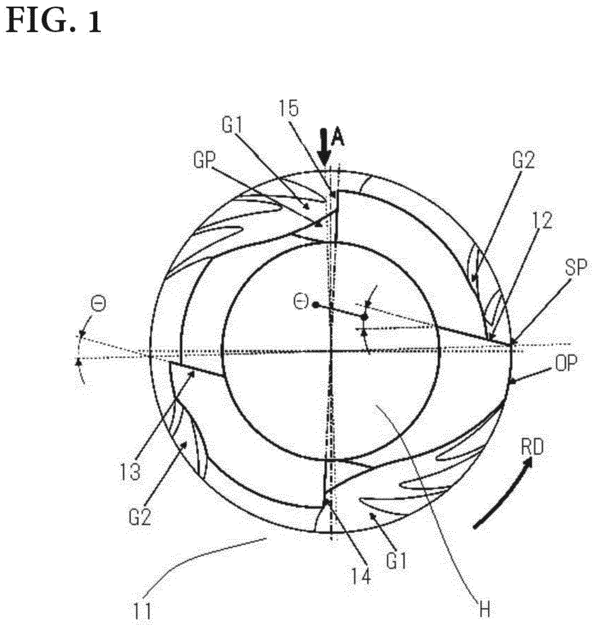

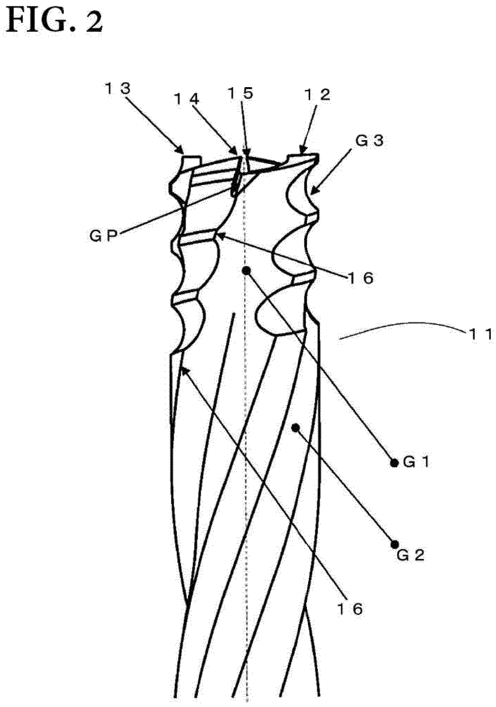

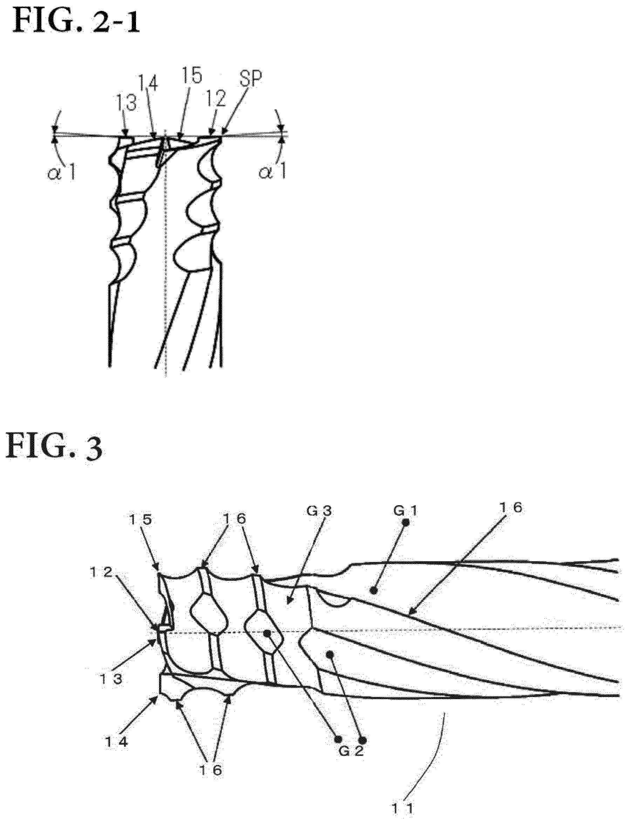

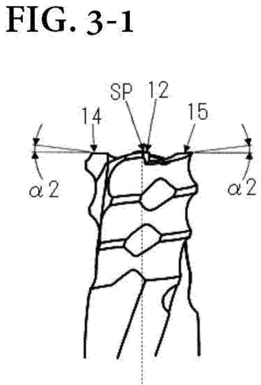

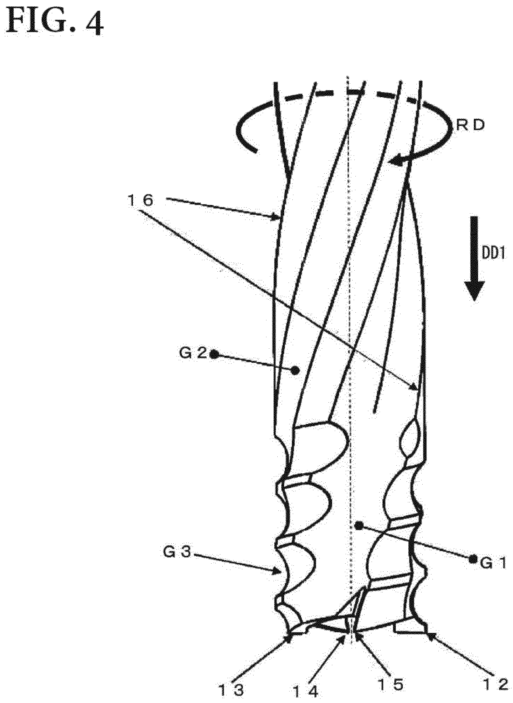

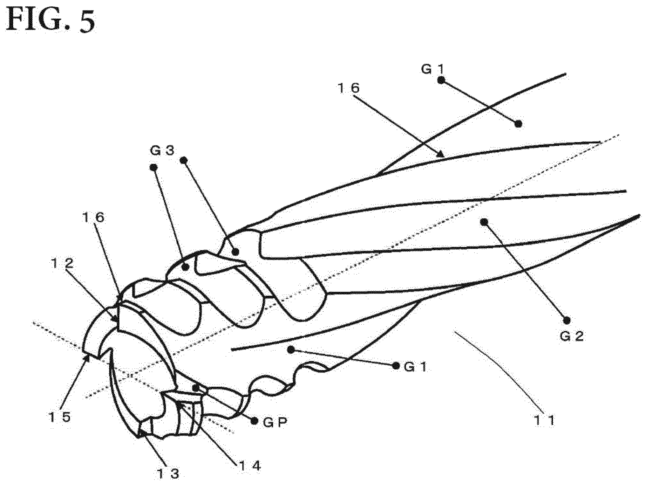

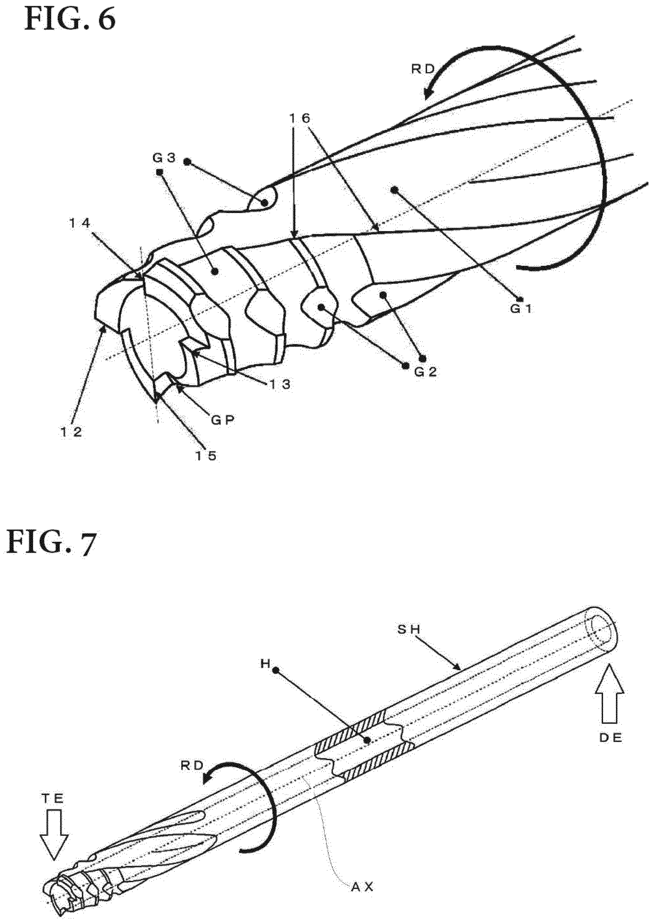

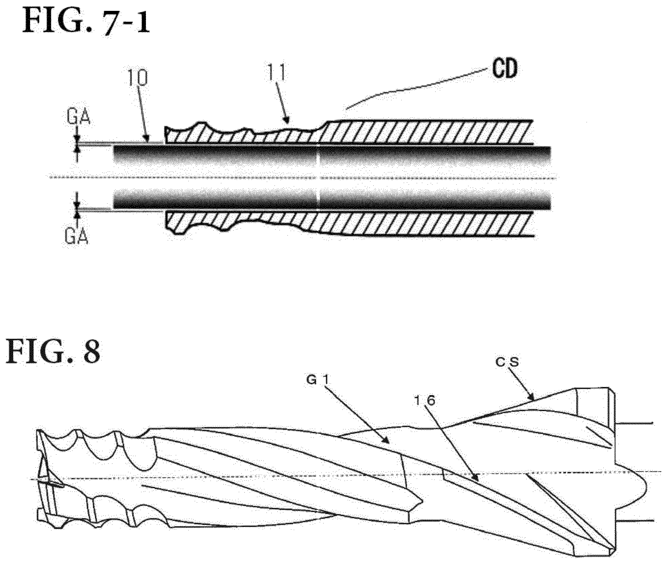

FIG. 1 is a front view of the tip end of the hollow drill bit for medical use according to Embodiment 1 of the present invention. FIG. 2 is a bottom view of the drill bit of FIG. 1. FIG. 2-1 is a bottom view of the drill bit of FIG. 1, and is an explanatory view of the tip end of the drill bit. FIG. 3 is a right side view of the drill bit of FIG. 1. FIG. 3-1 is a bottom view of the drill bit of FIG. 1, and is an explanatory view of the tip end of the drill bit. FIG. 4 is a top view of the drill bit of FIG. 1 when viewed from a direction perpendicular to and oblique to the main cutting edges. FIG. 5 is a perspective view of the drill bit in FIG. 1. FIG. 6 is a perspective view illustrating a different section from the perspective view of FIG. 5. FIG. 7 is a perspective view of the entirety of the drill bit of FIG. 1, from the tip to the distal tip.

Referring to FIG. 7, the hollow drill bit (11) of this embodiment is provided with a hollow portion (H) that penetrates the axial center (AX) from the tip end (TE) to the distal end (DE) in the axial direction.

Referring to FIGs. 1 and 7, the hollow drill bit (11) of this embodiment has four cutting edges (i.e. a main cutting edge 1 (12), a main cutting edge 2 (13), a sub cutting edge 1 (14), and a sub cutting edge 2 (15)) at the tip end (TE) section, and arc-shaped outer peripheral grooves (G3) are helically formed in a cylinder-shaped outer periphery (OP).

Further, chip discharging grooves (i.e. chip discharging grooves 1 (G1) and chip discharging grooves 2 (G2)) for discharging chips generated by the cutting edges mentioned above (i.e. the main cutting edge 1 (12), the main cutting edge 2 (13), the sub cutting edge 1 (14), and the sub cutting edge 2 (15)) during the use (during the bone-drilling operation) of the hollow drill bit (11) of this embodiment extend in a twisting manner in the direction from the tip end (TE) of the hollow drill bit (11) to the distal end (DE) (that is, toward the distal end (DE)).

Referring to FIG. 2 and 3, the chip discharging grooves (i.e. the chip discharging grooves 1 (G1) and the chip discharging grooves 2 (G2)) traverse the four cutting edges (i.e. the main cutting edge 1 (12), the main cutting edge 2 (13), the sub cutting edge 1 (14), and the sub cutting edge 2 (15)) and the outer peripheral grooves (G3).

In the hollow drill bit (11) of this embodiment, the helical direction of the outer peripheral grooves (G3) is same as the twisting direction of the chip discharging grooves (i.e. the chip discharging grooves 1 (G1) and the chip discharging grooves 2 (G2)), and the helical direction is the positive direction (in other words, the outer peripheral grooves (G3) are twisted to form a Z-shape when the tip end (TE) of the hollow drill bit (11) of this embodiment is viewed from above).

Referring to FIG. 1, the four cutting edges (i.e. the main cutting edge 1 (12), the main cutting edge 2 (13), the sub cutting edge 1 (14), and the sub cutting edge 2 (15)) have a linear shape inclined from the outer periphery (OP) toward the center of the hollow drill bit (11), and two cutting edges among the four cutting edges (i.e. the main cutting edge 1 (12), the main cutting edge 2 (13), the sub cutting edge 1 (14), and the sub cutting edge 2 (15)) that are formed at a distance from the center are main cutting edges (the main cutting edge 1 (12) and the main cutting edge 2 (13)), and the other two cutting edges that are formed 90° or more rearward of the main cutting edges (the main cutting edge 1 (12) and the main cutting edge 2 (13)) on a straight line through the center are sub cutting edges (the sub cutting edge 1 (14), and the sub cutting edge 2 (15)).

Further, referring to FIG. 1 and FIG. 2, the sub cutting edges (the sub cutting edge 1 (14), and the sub cutting edge 2 (15)) are provided with recesses referred to as gash pockets (GP) at the side of the rakes. These gash pockets (GP) are not provided for the drilling purpose. These gash pockets (GP) have an effect of preventing chips from entering the gap (GA) (See conventional FIG. 12 and FIG. 13, other than FIG. 7-1) between the hollow drill bit (11) and the small-diameter drill bit (D2) (FIG. 11 and FIG. 12) fitted in the hollow portion of the hollow drill bit (11) by keeping chips in the gash pockets (GP) in case where chips accumulate due to decreased cutting ability of the hollow drill bit (11) during the drilling operation. Such configuration has an advantageous effect of preventing the heat generation during the drilling process and achieving an improved cutting ability. In this embodiment, these gash pockets (GP) are provided starting from the inner periphery of the hollow drill bit (11) to the chip discharging grooves 1 (G1). These gash pockets (GP) do not have areas enlarging toward the inner periphery, unlike those in FIG. 13 and (a) and (b) of FIG. 14, but they are configured to have areas gradually enlarging from the ridge of the inner periphery toward the outer periphery.
Therefore, these gash pockets (GP) can prevent chips from occupying the gap (GA).

Also, in this embodiment, the two main cutting edges mentioned above comprise the main cutting edge 1 (12) and the main cutting edge 2 (13), where the main cutting edge 1 (12) and the main cutting edge 2 (13) are formed point-symmetrically in the similar manner.

The main cutting edge 1 (12) is formed starting from the cylinder-shaped portion of the outer periphery (OP). In contrast, the main cutting edge 2 (13) is formed starting from the outer peripheral grooves (G3) and is formed to be shorter than the main cutting edge 1 (12). As exemplified in FIG. 1, the main cutting edge 1 (12) and the main cutting edge 2 (13) have linear portions with an inclined angle (Θ) inclined away from the center. Having this inclined angle (Θ), the hollow drill bit (11) has a configuration that chips generated at the time of drilling the bone will not move in the direction toward the inner periphery of the hollow drill bit (11), but will move in the direction toward the outer periphery under the additional influence of centrifugal force when the hollow drill bit (11) in the present embodiments is rotated, and generates an advantageous effect of making it difficult for chips to move to the hollow portion (H) of the hollow drill bit (11). Further, the main cutting edge 1 (12) and the main cutting edge 2 (13) of the hollow drill bit (11) are configured such that the chip discharging grooves 1 (G1) and the chip discharging grooves 2 (G2) are not provided on the side of the rakes of the main cutting edge 1 (12) and the main cutting edge 2 (13).

The two sub cutting edges mentioned above comprise the sub cutting edge 1 (14) and the sub cutting edge 2 (15), where the sub cutting edge 1 (14) and the sub cutting edge 2 (15) are positioned 90° or more rearward of the main cutting edge 1 (12) and the main cutting edge 2 (13) relative to the rotational direction of the hollow drill bit (11), and where the sub cutting edge 1 (14) and the sub cutting edge 2 (15) are formed on a same straight line in a radial direction in the cylinder-shaped portion.

The sub cutting edge 1 (14) and the sub cutting edge 2 (15) of the hollow drill bit (11) in the embodiments do not serve in the drilling process, except the outer periphery on the side of the tip end. However, the sub cutting edge 1 (14) and the sub cutting edge 2 (15) are necessary elements to achieve a stable drilling process in a method of drilling helically, starting with the main cutting edge 1 (12).

Referring to FIGs. 1, 2, 2-1, 3 and 3-1, the angle α1 inclined toward the center of the main cutting edges (the main cutting edge 1 (12) and the main cutting edge 2 (13)) and the angle α2 inclined toward the center of the sub cutting edges satisfy the relationship of 0°<α1<α2. The angle α1 is set to be within the range of 0°<α 1 <5°, and the angle α2 is set to be equal to or greater than the angle α1 and less than 10°.

Preferably, the angle α1 and the angle α2 are set to satisfy the relationships of 1°<α1<3° and 5°<α2<7°.

Next, the chip discharging grooves (i.e. the chip discharging grooves 1 (G1) and the chip discharging grooves 2 (G2)) of the hollow drill bit (11) in the embodiments are described in details. The chip discharging grooves comprise the chip discharging grooves 1 (G1) provided on the side of the rakes of the sub cutting edges (the sub cutting edge 1 (14) and the sub cutting edge 2 (15)), and the chip discharging grooves 2 (G2) provided on the side surfaces on the side of the heels, as shown in FIG. 1. The chip discharging grooves 1 (G1) are gently connected in the direction from the side of the heels of the main cutting edges (the main cutting edge 1 (12) and the main cutting edge 2 (13)) at the tip end (TE) of the hollow drill bit (11) to the distal end (DE) of the hollow drill bit (11), and are connected to the helical outer peripheral grooves (G3) continuing from the side of the rakes of the main cutting edges (the main cutting edge 1 (12) and the main cutting edge 2 (13)) with a twist toward the side of the shank, which is at the distal end (DE) of the drill bit. The chip discharging grooves 2 (G2) have the same helix direction and the same helix angle as the chip discharging grooves 1 (G1).

In this embodiment, the main cutting edges (the main cutting edge 1 (12) and the main cutting edge 2 (13)) are connected to the helical outer peripheral grooves (G3) continuing to the side of the rakes of the main cutting edges, thereby providing an advantageous effect of allowing chips to be smoothly discharged. The outer peripheral grooves (G3) are formed helically and connected to the side of the rake of the main cutting edge 1 (12), which allows chips generated during the drilling process performed mainly by the main cutting edge 1 (12) to be sent, through the helical outer peripheral grooves (G3) continuing to the side of the rake of the main cutting edge 1 (12), to the chip discharging grooves (G1) and be discharged therefrom. Therefore, chips are discharged mainly from the chip discharging grooves 1 (G1) connected to the side of the rake of the sub cutting edge 1 (14), through the outer peripheral grooves (G3) connected from the side of the rake of the main cutting edge 1 (12). The chip discharging grooves are not provided on the side of the rakes of the main cutting edges (the main cutting edge 1 (12) and the main cutting edge 2 (13)). The helical outer peripheral grooves (G3) on the side of the outer periphery of the cutting edges serve as chip discharging grooves.

Further, the chip discharging grooves 2 (G2) shallower and smaller than the chip discharging grooves 1 (G1) are provided starting from the side surfaces of the side of the heels of the sub cutting edges (the sub cutting edge 1 (14) and the sub cutting edge 2 (15)). As mentioned above, the chip discharging grooves 2 (G2) have the same rake direction and the same rake angle as the chip discharging grooves 1 (G1), and are provided as complementary chip discharging grooves. The chip discharging grooves 2 (G2) are provided on the side surfaces on the side of the heels of the sub cutting edges (the sub cutting edge 1 (14) and the sub cutting edge 2 (15)) and forward of the main cutting edges (the main cutting edge 1 (12) and the main cutting edge 2 (13)), and are arranged at a location synchronized with the location where chips are discharged during the drilling process by the inner periphery of the main cutting edges (the main cutting edge 1 (12) and the main cutting edge 2 (13)).

The helical outer peripheral grooves (G3), the chip discharging grooves 1 (G1), and the chip discharging grooves 2 (G2), which are characteristics of the outer periphery of the hollow drill bit (11) in the embodiments, are provided to reduce the contact area between the outer periphery and the bone, for the hollow drill bit (11) in the embodiments has a configuration with four cutting edges (i.e. the main cutting edge 1 (12), the main cutting edge 2 (13), the sub cutting edge 1 (14), and the sub cutting edge 2 (15)). Therefore, the hollow drill bit (11) in this embodiment produces an excellent effect of reducing the friction due to the contact and suppressing the rise in temperature.

The hollow drill bit (11) of the embodiments is provided with the above-mentioned configuration, and the drilling process starts with the outer periphery of the main cutting edge 1 (12) being brought into contact with the bone, and then the tip of each outer periphery being brought into contact with the bone in the order of the sub cutting edge 1 (14), the main cutting edge 2 (13), and the sub cutting edge 2 (15), and subsequently the main cutting edge 1 (12) and the main cutting edge 2 (13). A hole with a diameter corresponding to the diameter of the hollow drill bit (11) is achieved by the drilling process by the main cutting edge 1 (12). The reference numeral (SP) in FIG. 1 indicates the starting point of the drilling process.

The structure from the tip end to the chip discharging grooves 1 (G1) and the chip discharging grooves 2 (G2) of the hollow drill bit (11) according to this embodiment is illustrated in FIG. 1 to FIG. 6, and has, toward the shank at the side of the distal end (DE), a pipe shape (or tube shape) with the same diameter as the drill diameter of the hollow drill bit (11), as shown in FIG. 7, or a thinner pipe shape (or tube shape) than the drill diameter, and an entire length set depending on the type of the operation.

### [Embodiment 2]

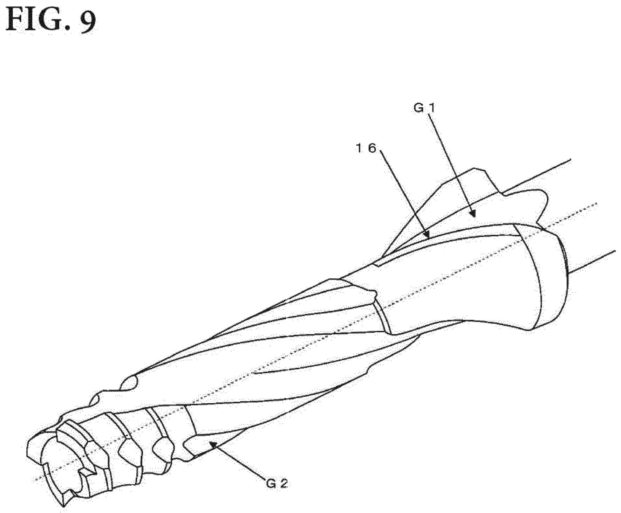

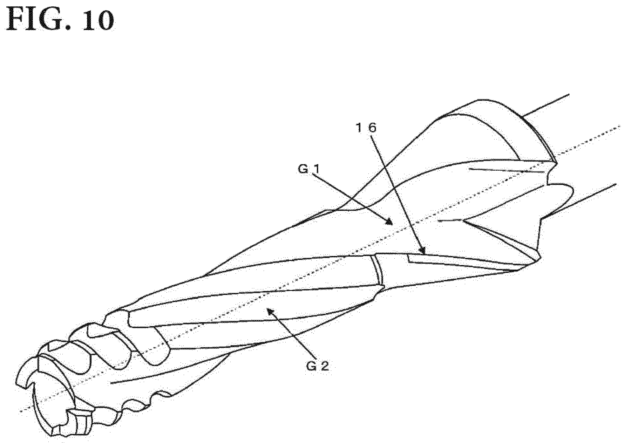

FIG. 8 is a side view of an example of a hollow drill bit for medical use according to Embodiment 2. FIG. 9 is a perspective view of the drill bit of FIG. 8. FIG. 10 is a perspective view illustrating a different section of the drill bit from the perspective view of FIG. 9.

The hollow drill bit (11) according to Embodiment 2 is provided with counter sinks (CS) near the rearward ends of the chip discharging grooves 1 (G1) to simultaneously perform the burr removal and chamfering process after the drilling process by the drill bit.
It should be understood that the hollow drill bit (11) according to this embodiment adopts the same structure as that of the above-mentioned Embodiment 1, except the counter sinks (CS).

### [Embodiment 3]

Referring to FIG. 7 and FIG. 7-1, the hollow drill bit (11) of the composite drill bit for medical use (CD) according to Embodiment 3 adopts the same configuration as that of the above-mentioned Embodiment 1 or Embodiment 2, and comprise a small-diameter drill bit (10) fitted into the hollow portion (H) (See FIG. 7) of the hollow drill bit (11) such that the small-diameter drill bit (10) does not rotate together with the hollow drill bit (11). The hollow drill bit (11) may adopt a small-diameter drill bit described in the above-mentioned conventional art or another well-known small-diameter drill bit as the small-diameter drill bit (10).

### [Embodiment 4]

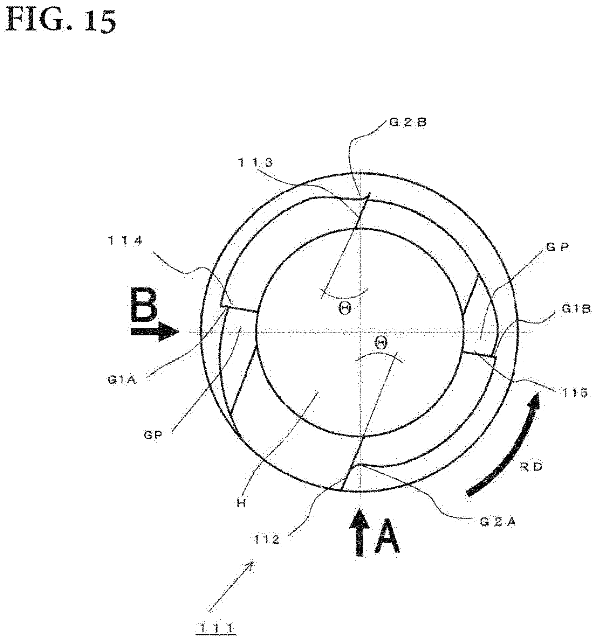

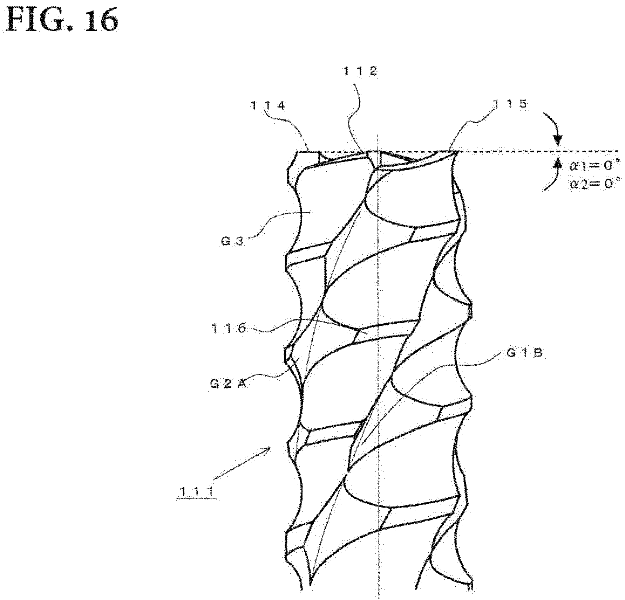

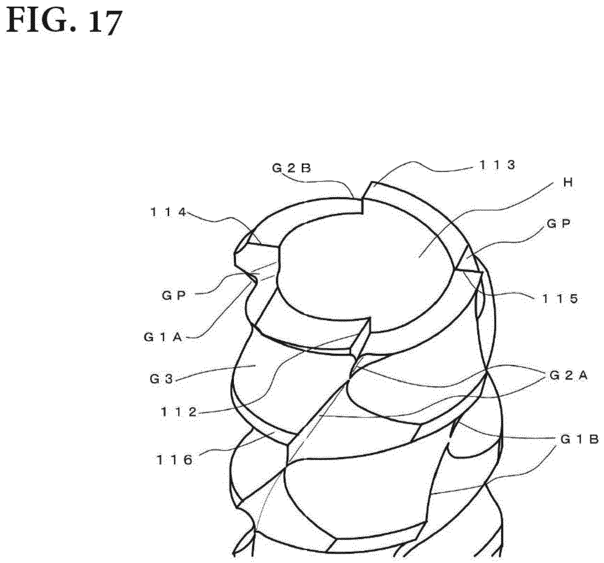

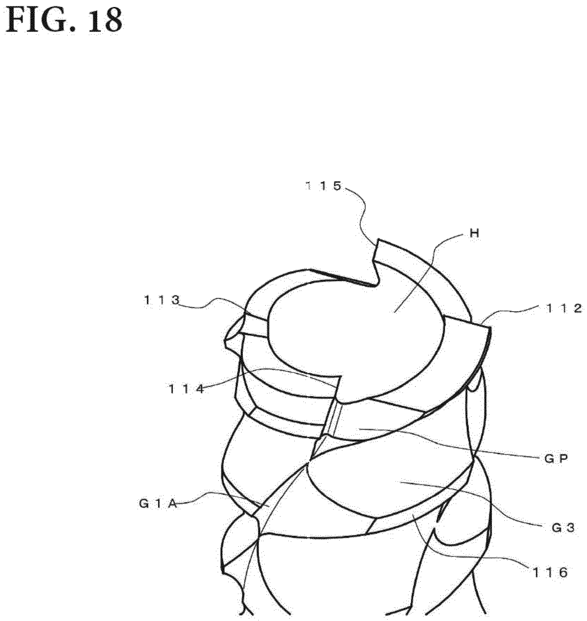

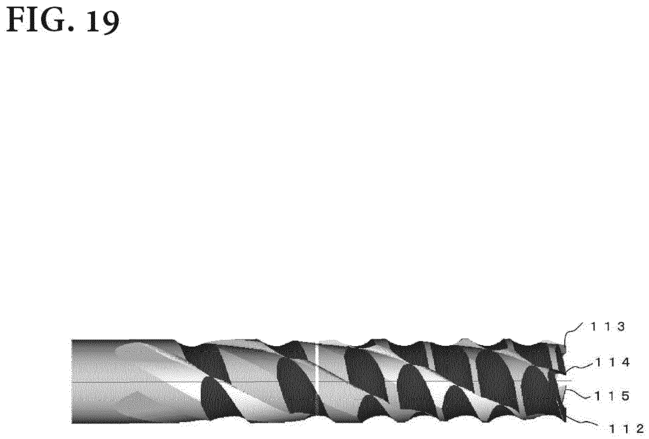

FIG. 15 is a front view of the tip end of the hollow drill bit for medical use according to an embodiment of the present invention. FIG. 16 is a bottom view of the drill bit of FIG. 15. FIG. 17 is a perspective view of the drill bit of FIG. 15 when the tip end is viewed from a direction substantially parallel to and oblique to the main cutting edges. FIG. 18 is a perspective view of the drill bit of FIG. 15 when the tip end is viewed from a direction substantially parallel to and oblique to the sub cutting edges. FIG. 19 is a left side view of the drill bit of FIG. 15.

It was assumed that the hollow drill bit according to [Embodiment 1] mentioned above has a structure in which a certain thickness can be ensured. Therefore, large chip discharging grooves 1 (G1) were arranged rearward of the main cutting edge 1 (12) and the main cutting edge 2 (13) (and forward of the sub cutting edges) (relative to the rotational direction), and the chip discharging grooves 2 (G2) were complementarily provided in the outer periphery of the drill.

However, with consideration of a case where the same structure is manufactured with a thinner thickness and the drill bit does not have the chip discharging effect or the drilling ability as anticipated, the drill bit has a structure in which chips are not discharged from the chip discharging grooves like in [Embodiment 1], but from the outer peripheral grooves (G3) helically provided in the outer periphery, and in which the angles of the main cutting edge 1 (112) and the main cutting edge 2 (113), and the angles of the sub cutting edge 1 (114) and the sub cutting edge 2 (115) are set to be parallel so that all of the four cutting edges are able to drill, and, based on such structure, the sub cutting edges 1 and 2 have rake angles set and have shapes for performing most of the drilling process.

Conventional chip discharging grooves are provided as grooves forming outer peripheral cutting edges (G1A, G1B, G2A, G2B) to form outer peripheral cutting edges (116) and the depth of the grooves is set to be equivalent to that of the outer peripheral grooves (G3) or shallower than that of the outer peripheral grooves (G3) to allow chips to be led by the outer peripheral grooves (G3) through the helical locus to the rearward end of the drill bit and then discharged therefrom.

Referring to FIG. 15, FIG. 17 and FIG. 18, the hollow drill bit (111) in this embodiment has four cutting edges (i.e. a main cutting edge 1 (112), a main cutting edge 2 (113), a sub cutting edge 1 (114), and a sub cutting edge 2 (115)) at the tip end (TE) section, and arc-shaped outer peripheral grooves (G3) helically formed in a cylindrical-shaped outer periphery (OP).

Further, the grooves forming outer peripheral cutting edges (i.e. the groove forming an outer peripheral cutting edge 1A (G1A), the groove forming an outer peripheral cutting edge 1B (G1B), the groove forming an outer peripheral cutting edge 2A (G2A), and the grooves forming an outer peripheral cutting edge 2B (G2B)) for allowing chips generated by the cutting edges mentioned above (i.e. the main cutting edge 1 (112), the main cutting edge 2 (113), the sub cutting edge 1 (114), and the sub cutting edge 2 (115)) during the use of the hollow drill bit (11) of this embodiment (during the bone-drilling operation) to be led to the outer peripheral grooves (G3) while forming the outer peripheral cutting edges, extend in a twisting manner in the direction from the tip end (TE) of the hollow drill bit (111) to the distal end (DE) (i.e. toward the distal end (DE)).

Referring to FIG. 16, the grooves forming outer peripheral cutting edges (i.e. the groove forming an outer peripheral cutting edge 1A (G1A), the groove forming an outer peripheral cutting edge 1B (G1B), the groove forming an outer peripheral cutting edge 2A (G2A), and the grooves forming an outer peripheral cutting edge 2B (G2B)) traverse the four cutting edges (i.e. the main cutting edge 1 (112), the main cutting edge 2 (113), the sub cutting edge 1 (114), and the sub cutting edge 2 (115)) and the outer peripheral grooves (G3).

In the hollow drill bit (11) of this embodiment, the helical direction of the outer peripheral grooves (G3) is same as the twisting direction of the grooves forming outer peripheral cutting edges (i.e. the groove forming an outer peripheral cutting edge 1A (G1A), the groove forming an outer peripheral cutting edge 1B (G1B), the groove forming an outer peripheral cutting edge 2A (G2A), and the grooves forming an outer peripheral cutting edge 2B (G2B)), and the helical direction is the positive direction (in other words, the outer peripheral grooves (G3) are twisted to form a Z-shape when the tip end (TE) of the hollow drill bit (111) of this embodiment is viewed from above).

Referring to FIG. 16, the four cutting edges (i.e. the main cutting edge 1 (112), the main cutting edge 2 (113), the sub cutting edge 1 (114), and the sub cutting edge 2 (115)) have a horizontal linear shape from the outer periphery (OP) of the hollow drill bit (111) toward the center. The angle α1 of the main cutting edges (the main cutting edge 1 (112) and the main cutting edge 2 (113)) inclined toward the center, and the angle α2 of the sub cutting edge 1 (114) and the sub cutting edge 2 (115) inclined toward the center satisfy the relationship of α1=α2=0. Two cutting edges among the four cutting edges (i.e. the main cutting edge 1 (112), the main cutting edge 2 (113), the sub cutting edge 1 (114), and the sub cutting edge 2 (115)) that are formed at a distance from the center are the main cutting edges (the main cutting edge 1 (112) and the main cutting edge 2 (113)), and the angle between the main cutting edge 1 (112) and the sub cutting edge 1 (114), and the angle between the main cutting edge 2 (113) and the sub cutting edge 2 (115) may be from 90° to 100°, respectively.

Further, referring to FIG. 15, FIG. 17, and FIG. 18, the sub cutting edges (the sub cutting edge 1 (114) and the sub cutting edge 2 (115)) are provided with recesses referred to as gash pockets (GP) at the side of the rakes. These gash pockets (GP) are provided to enhance the chip-discharge ability and the drilling ability, and the rakes are formed starting from the gash pockets (GP) toward the edges of the sub cutting edge 1

(114) and the sub cutting edge 2 (115). Chips are discharged from the gash pockets (GP) to the outer peripheral grooves through the grooves forming outer peripheral cutting edges directly below the gash pockets (GP), which produces an effect of preventing chips from entering the gap (GA) (See FIG. 12 and FIG. 13 of a conventional art, in addition to FIG. 7-1) between the hollow drill bit (111) and the small-diameter drill bit (D2) (See FIG. 11 and FIG. 12) fitted into the hollow portion of the hollow drill bit (111). This configuration produces an advantageous effect of preventing the heat generation during the drilling process and, as a result, achieving an improved cutting ability. In this embodiment, these gash pockets (GP) are provided starting from the inner periphery of the hollow drill bit (111) to the grooves forming outer peripheral cutting edges 1A (G1A) and 2A (G2A). These gash pockets (GP) do not have areas enlarging toward the inner periphery, unlike those in FIG. 13 and (a) and (b) of FIG. 14, but they are configured to have areas gradually enlarging from the ridge of the inner periphery toward the outer periphery. Therefore, these gash pockets (GP) can prevent chips from occupying the gap (GA).

Also, in this embodiment, the two main cutting edges mentioned above comprise the main cutting edge 1 (112) and the main cutting edge 2 (113), where the main cutting edge 1 (112) and the main cutting edge 2 (113) are formed point-symmetrically in the similar manner.

The main cutting edge 1 (112) is formed starting from the cylinder-shaped portion of the outer periphery (OP). In contrast, the main cutting edge 2 (113) is formed starting from the outer peripheral grooves (G3), and is formed to be shorter than the main cutting edge 1 (112). The main cutting edge 1 (112) serves to allow the tip end to penetrate into the bone and allow the drilling locus to form a cylinder shape during the drilling process of the hollow drill bit.

Therefore, the main cutting edge 1 prevents the hole from having a thread shape.

As exemplified in FIG. 1, the main cutting edge 1 (112) and the main cutting edge 2 (113) have linear portions with an inclined angle (Θ) inclined away from the center. Having this inclined angle (Θ), the hollow drill bit (111) in this embodiment is configured to allow chips generated during the bone-drilling process not to move in the direction toward the inner periphery of the foregoing hollow drill bit (111), but to move in the direction toward the outer periphery under the additional influence of centrifugal force when the hollow drill bit (111) in this embodiment is rotated, which generates an advantageous effect of making it difficult for chips to move to the hollow portion (H) of the hollow drill bit (111).

The two sub cutting edges mentioned above comprise the sub cutting edge 1 (114) and the sub cutting edge 2 (115), where the sub cutting edge 1 (114) and the sub cutting edge 2 (115) are positioned 90° or more rearward of the main cutting edge 1 (112) and the main cutting edge 2 (113) relative to the rotational direction of the foregoing hollow drill bit (111), respectively, and where the sub cutting edge 1 (114) and the sub cutting edge 2 (115) are formed on a same straight line in a radial direction in the cylinder-shaped portion. The cutting edges, including the main cutting edges and the sub cutting edges, may be located equi-angularly at 90° at four locations in total. Alternatively, in consideration of the creation of the gash pockets, the angles between the main cutting edges and the sub cutting edges may be set to 90°<=100°.

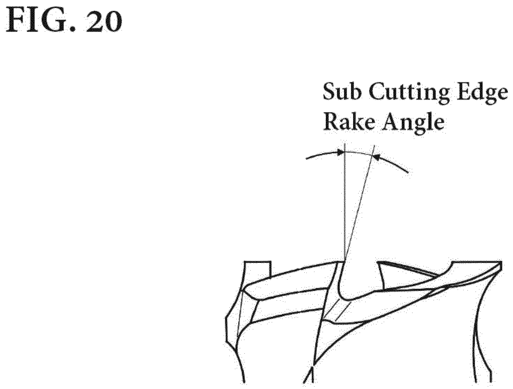

As shown in FIG. 20, the rake angles of the main cutting edge 1 (112) and the main cutting edge 2 (113) as well as the rake angles of the sub cutting edge 1 (114) and the sub cutting edge 2 (115) are not particularly limited. However, since a negative rake angle will cause significant damage to the cutting ability, the rake angles are set to 0° <= the rake angles of the main cutting edges < the rake angles of sub cutting edges.

The angles of the main cutting edges (112) and (113) as well as the angles of the sub cutting edges (114) and (115) of the hollow drill bit (111) in this embodiment are set to be horizontal to allow all four cutting edges to have the cutting ability. Then, the sub cutting edges 1 and 2 have rake angles set and have shapes for performing most of the drilling process.

Next, the grooves forming outer peripheral cutting edges (i.e. the grooves forming outer peripheral cutting edges 1A (G1A), the grooves forming outer peripheral cutting edges 1B (G1B), the grooves forming outer peripheral cutting edges 2A (G2A), and the grooves forming outer peripheral cutting edges 2B (G2B)) of the hollow drill bit (111) in this embodiment is described in details. As shown in FIG. 17, the grooves forming outer peripheral cutting edges comprise the grooves forming outer peripheral cutting edges 1A (G1A) and the grooves forming outer peripheral cutting edges 1B (G1B) provided at the side of the rakes of the sub cutting edges (the sub cutting edge 1 (114) and the sub cutting edge 2 (115)), and the grooves forming outer peripheral cutting edges 2A (G2A) and the grooves forming outer peripheral cutting edges 2B (G2B) provided on the side surfaces at the side of the heels. The grooves forming outer peripheral cutting edges 1A (G1A) and the grooves forming outer peripheral cutting edges 1B (G1B) are gently connected in the direction from the side of the heels of the main cutting edges (the main cutting edge 1 (112) and the main cutting edge 2 (113)) at the tip end (TE) of the hollow drill bit (111) to the distal end (DE) of the hollow drill bit (111), and are connected to the helical outer peripheral grooves (G3) continuing to the side of the rakes of the main cutting edges (the main cutting edge 1 (112) and the main cutting edge 2 (113)) with a twist toward the side of the shank, which is at the distal end (DE) of the drill bit. The grooves forming outer peripheral cutting edges 2A and 2B ((G2A) and (G2B)) have the same helix direction and the same helix angle as the grooves forming outer peripheral cutting edges 1A and 1B ((G1A) and (G2A)).

The grooves forming outer peripheral cutting edges have a depth set to be equivalent to the depth of the outer peripheral grooves (G3) or shallower than the depth of the outer peripheral grooves (G3). Large bottom surfaces are not formed. Instead, bottom surfaces with a very small diameter are formed gradually toward the rearward of the outer peripheral cutting edges. The grooves may be provided with a small volume and also with a gentle helix angle from about 5° to about 30°, for these grooves do not serve to allow chips to be discharged.

In this embodiment, most of the drilling process is performed by the sub cutting edge 1 (114) and the sub cutting edge 2 (115), and a cylinder shape is formed by the main cutting edge 1 (112), with the outer periphery (OP) of the cutting edge being corresponding to the drill diameter. The main cutting edge 2 (113) is hardly involved in the drilling process, but is present as an essential element to allow the four cutting edges to perform stable drilling during the drilling process. This produces an advantageous effect of allowing chips generated by the cutting with the sub cutting edge 1 (114), the sub cutting edge 2 (115), and the main cutting edge 1 (112) to be smoothly discharged from the outer peripheral grooves (G3) via the gash pockets (GP), the grooves forming outer peripheral cutting edges 1A (G1A), the grooves forming outer peripheral cutting edges 1B (G1B), and the grooves forming outer peripheral cutting edges 2A (G2A), for the helical outer peripheral groove (G3) are connected directly below the grooves forming outer peripheral cutting edges 2A (G2A).

When the hollow drill bit has a small diameter or a thin thickness, the chip discharging grooves (G1) like in [Embodiment 1] may not be provided. In such case, this embodiment can solve the problem.

The helical peripheral grooves (G3), the grooves forming outer peripheral cutting edges 1A (G1A), the grooves forming outer peripheral cutting edges 1B (G1B), the grooves forming outer peripheral cutting edges 2A (G2A), and the grooves forming outer peripheral cutting edges 2B (G2B), which are characteristics of the outer periphery of the hollow drill bit (111) in this embodiment, are provided to reduce the contact area between the outer periphery and the bone, for the configuration with the four cutting edges (i.e. the main cutting edge 1 (112), the main cutting edge 2 (113), the sub cutting edge 1 (114), and the sub cutting edge 2 (115)) increases the opportunity of contact between the outer peripheral cutting edges (116) and the bone. Therefore, the hollow drill bit (111) in this embodiment produces an excellent effect of reducing the friction due to the contact and suppressing the rise in temperature.

The hollow drill bit (111) in this embodiment is provided with the configuration mentioned above, and the main cutting edge 1 (112) is used to penetrate into the bone (to ensure that a hole is made with a diameter as large as the drill diameter), and then the sub cutting edges (the sub cutting edge 1 (114) and the sub cutting edge 2 (115)) are used to perform most of the drilling process, and the main cutting edge 2 (113) is used to complement the main cutting edge 1 (112) as it penetrates into the bone.

The structure from the tip end to the grooves forming outer peripheral cutting edges 1A (G1A), the grooves forming outer peripheral cutting edges 1B (G1B), the grooves forming outer peripheral cutting edges 2A (G2A), and the grooves forming outer peripheral cutting edges 2B (G2B) of the hollow drill bit (111) according to this embodiment is illustrated in FIG. 15 to FIG. 20, and has, toward the shank at the side of the distal end (DE), a pipe shape (or tube shape) with the same diameter as the drill diameter of the hollow drill bit (111), as shown in FIG. 7, or a thinner pipe shape (or tube shape) than the drill diameter, and an entire length set depending on the type of the operation. For example, FIG. 19 shows a structure on the assumption that a deep hole will be made. In a case where a deep hole will be made, the grooves forming outer peripheral cutting edges are provided throughout the long entire length, and, at the same time, the helical outer peripheral grooves are provided on a close pitch at the side of the tip end, and may be provided on an increased pitch at the side of the rearward end for this side is hardly involved in the drilling process.

### [Industrial applicability]

The hollow drill bit for medical use of the present invention is to design a novel tip end shape that performs most of the drilling process so as to prevent chips from entering the gap between the hollow drill bit and the small-diameter drill bit fitted into the hollow portion of the hollow drill bit as well as to provide a structure of the outer periphery so as to allow chips to be discharged toward the outer periphery and prevent them from entering the gap portion.

### EXPLANATIONS OF REFERENCES

- 10: Small-diameter drill bit
- 11: Hollow drill bit
- 12: Main cutting edge 1
- 13: Main cutting edge 2
- 14: Sub cutting edge 1
- 15: Sub cutting edge 2
- 16: Outer peripheral cutting edges
- 111: Hollow drill bit
- 112: Main cutting edge 1
- 113: Main cutting edge 2
- 114: Sub cutting edge 1
- 115: Sub cutting edge 2
- 116: Outer peripheral cutting edges
- AX: Axial center
- CD: Compound drill bit
- D1: Conventional hollow drill bit
- D2: Conventional small-diameter drill bit
- DD1: Drilling direction
- DE: Distal end
- G1: Chip discharging grooves 1
- G2: Chip discharging grooves 2
- G1A: Grooves forming outer peripheral cutting edges 1A
- G1B: Grooves forming outer peripheral cutting edges 1B
- G2A: Grooves forming outer peripheral cutting edges 2A
- G2B: Grooves forming outer peripheral cutting edges 2B
- G3: Helical outer peripheral groove
- GA: Gap
- GP: Gash pocket
- H: Hollow portion
- OP: Outer periphery
- RD: Rotational direction
- SP: Starting point of drilling process
- TE: Tip end
- Θ: Inclined angle of the main cutting edge
- α1: Angle inclined toward the center of the main cutting edge
- α2: Angle inclined toward the center of the sub cutting edge
- CS: Counter sink

## Claims

1. A hollow drill bit (11) for medical use comprising
a hollow portion (H) that penetrates an axial center in an axial direction from a tip end (TE) to a distal end (DE); and
four cutting edges (12, 13, 14, 15) at a section of the tip end (TE),
wherein arc-shaped outer peripheral grooves (G1, G2, G3) are helically formed in a cylinder-shaped outer periphery (OP),
wherein a helical direction of the outer peripheral grooves (G1, G2, G3) is a forward direction, wherein the four cutting edges (12, 13, 14, 15) have a linear shape inclined from the outer periphery (OP) to a center of the hollow drill bit,
wherein two cutting edges (12, 13) among the four cutting edges that are formed at a distance from the center are main cutting edges,
wherein other two cutting edges (14, 15) that are formed 90° or more rearward from the main cutting edges (12, 13) on a straight line through the center are sub cutting edges (14, 15), wherein the sub cutting edges (14, 15) are provided with gash pockets (GP).

2. The hollow drill bit (11) for medical use according to Claim 1, wherein chip discharging grooves (G1, G2) for discharging chips generated by the cutting edges (12, 13, 14, 15) during use of the hollow drill bit (11) extend in a twisting manner in a direction from the tip end (TE) to the distal end (DE) of the hollow drill bit (11),
wherein the chip discharging grooves traverse the four cutting edges and the outer peripheral grooves, and
wherein the helical direction of the outer peripheral grooves is same as a twisting direction of the chip discharging grooves.

3. The hollow drill bit (11) for medical use according to Claim 1 or 2, wherein the two main cutting edges (12, 13) consist of a main cutting edge 1 (12) and a main cutting edge 2 (13),
wherein the main cutting edge 1 (12) and the main cutting edge 2 (13) are formed point-symmetrically in a same manner,
wherein the main cutting edge 1 (12) is formed starting from a cylinder-shaped portion of the outer periphery (OP), and
wherein the main cutting edge 2 (13) is formed starting from the outer peripheral grooves (G1, G2) and is formed to be shorter than the main cutting edge 1 (12).

4. The hollow drill bit (11) for medical use according to any one of Claims 1 to 3, wherein the two sub cutting edges (14, 15) consist of a sub cutting edge 1 (14) and a sub cutting edge 2 (15),
wherein the sub cutting edge 1 (14) and the sub cutting edge 2 (15) are positioned 90° or more rearward of the main cutting edge 1 (12) and the main cutting edge 2 (13) relative to a rotational direction of the hollow drill bit (11), and
wherein the sub cutting edge 1 (14) and the sub cutting edge 2 (15) are formed on a same straight line in a radial direction in the cylinder-shaped portion.

5. The hollow drill bit (11) for medical use according to any one of Claims 1 to 4, wherein an angle α1 of the main cutting edges (12, 13) inclined toward the center and an angle α2 of the sub cutting edges (14, 15) inclined toward the center satisfy a relationship of 0°<α1<α2.

6. The hollow drill bit (11) for medical use according to any one of Claims 1 to 5, wherein an inclined angle (Θ) made by the main cutting edge 1 (12) and the main cutting edge 2 (13) is formed in a direction in which chips are discharged to a side of the outer periphery (OP) due to rotation of the hollow drill bit (11), and as a result, chips generated by drilling process of the main cutting edge 1 (12) are discharged from a side of a rake of the main cutting edge 1 (12) to chip discharging grooves 1 (G1) via outer peripheral grooves (G3),
wherein the chip discharging grooves (G1, G2) comprise chip discharging grooves 1 (G1) provided at the side of the rakes of the sub cutting edges (14, 15) and chip discharging grooves 2 (G2) provided on side surfaces at a side of heels,
wherein the chip discharging grooves 1 (G1) are gently connected in the direction from the side of the heels of the main cutting edges (12, 13) at the tip end (TE) of the hollow drill bit (11) to the distal end (DE) of the hollow drill bit (11), and are connected to the helical outer peripheral grooves (G1, G2) continuing to the side of the rakes of the main cutting edges (12, 13) with a twist toward the side of the shank, which is at the distal end (DE) of the drill bit (11), and
wherein the chip discharging grooves 2 (G2) have a same helix direction and a same helix angle as the chip discharging grooves 1 (G1).

7. A composite drill bit comprising a hollow drill bit (11) according to any one of Claims 1 to 6 and small-diameter drill bit fitted into the hollow portion (H) of said hollow drill bit (11).

8. The hollow drill bit (11) for medical use according to Claim 1, wherein grooves forming outer peripheral cutting edges (G1A) with a depth equivalent to or shallower than a depth of the outer peripheral grooves (G3) extend in a twisting manner in the direction from the tip end (TE) to the distal end (DE) of the hollow drill bit (11),
wherein the grooves forming outer peripheral cutting edges (G1B) transverse the four cutting edges (12, 13, 14, 15) and the outer peripheral grooves (G3), and
wherein the helical direction of the outer peripheral grooves (G3) is same as the twisting direction of the grooves forming outer peripheral cutting edges (G1A).

9. The hollow drill bit (11) for medical use according to Claim 8, wherein two cutting edges (112, 113) among the four cutting edges (112, 113, 114, 115) that are formed at a distance from the center are main cutting edges (112, 113), and other two cutting edges (114, 115) that are formed 90° or more rearward from the main cutting edges (112, 113) on a straight line through the center are sub cutting edges (114, 115),
wherein an angle α1 of the main cutting edges (112, 113) inclined toward the center and an angle α2 of the sub cutting edges (114, 115) inclined toward the center satisfy a relationship of α1=α2=0, wherein the sub cutting edges (114, 115) are provided with gash pockets, and
wherein the helical direction of the outer peripheral grooves (G3) is a positive direction.

10. The hollow drill bit (11) for medical use according to Claim 8 or 9, wherein the two main cutting edges (112, 113) consist of a main cutting edge 1 (112) and a main cutting edge 2 (113),
wherein the main cutting edge 1 (112) and the main cutting edge 2 (113) are formed point-symmetrically in a same manner,
wherein the main cutting edge 1 (112) is formed starting from a cylinder-shaped portion of the outer periphery (OP), and
wherein the main cutting edge 2 (113) is formed starting from the outer peripheral grooves (G3) and is formed to be shorter than the main cutting edge 1 (112).

11. The hollow drill bit (11) for medical use according to any one of Claims 8 to 10, wherein the two sub cutting edges (114, 115) consist of a sub cutting edge 1 (114) and a sub cutting edge 2 (115),
wherein the sub cutting edge 1 (114) and the sub cutting edge 2 (115) are positioned 90° or more rearward of the main cutting edge 1 (112) and the main cutting edge 2 (113) relative to a rotational direction of the hollow drill bit (11), and
wherein the sub cutting edge 1 (114) and the sub cutting edge 2 (115) are formed on a same straight line in a radial direction in the cylinder-shaped portion.

12. A composite drill bit comprising a hollow drill bit (11) according to any one of Claims 8 to 11 and a small-diameter drill bit fitted into the hollow portion (H) of said hollow drill bit (11).

## Patentansprüche

1. Hohlbohreinsatz (11) für medizinische Verwendung, umfassend
einen Hohlteil (H), der ein axiales Zentrum in einer axialen Richtung von einem Spitzenende (TE) bis zu einem distalen Ende (DE) durchdringt; und
vier Schneidkanten (12, 13, 14, 15) an einem Abschnitt des Spitzenendes (TE),
wobei bogenförmige Außenumfangsrillen (G1, G2, G3) spiralförmig in einem zylinderförmigen Außenumfang (OP) ausgebildet sind,
wobei eine Spiralrichtung der Außenumfangsrillen (G1, G2, G3) in Vorwärtsrichtung verläuft,
wobei die vier Schneidkanten (12, 13, 14, 15) eine lineare Form aufweisen, die vom Außenumfang (OP) zu einem Zentrum des Hohlbohreinsatzes geneigt ist,
wobei zwei Schneidkanten (12, 13) unter den vier Schneidkanten, die in einem Abstand vom Zentrum ausgebildet sind, Hauptschneidkanten sind,
wobei die anderen beiden Schneidkanten (14, 15), die um 90° oder mehr hinter den Hauptschneidkanten (12, 13) auf einer durch das Zentrum verlaufenden geraden Linie ausgebildet sind, Nebenschneidkanten (14, 15) sind, wobei für die Nebenschneidkanten (14, 15) Spantaschen (GP) versehen sind.

2. Hohlbohreinsatz (11) für medizinische Verwendung nach Anspruch 1, wobei Späneabführrillen (G1, G2) zum Abführen von Spänen, die durch die Schneidkanten (12, 13, 14, 15) während der Verwendung des Hohlbohreinsatzes (11) erzeugt werden, sich verdrehend in einer Richtung von dem Spitzenende (TE) zum distalen Ende (DE) des Hohlbohreinsatzes (11) erstrecken,
wobei die Späneabführrillen die vier Schneidkanten und die Außenumfangsrillen durchqueren und
wobei die Spiralrichtung der Außenumfangsrillen dieselbe ist wie die Verdrehungsrichtung der Späneabführrillen.

3. Hohlbohreinsatz (11) für medizinische Verwendung nach Anspruch 1 oder 2, wobei die beiden Hauptschneidkanten (12, 13) aus einer Hauptschneidkante 1 (12) und einer Hauptschneidkante 2 (13) bestehen,
wobei die Hauptschneidkante 1 (12) und die Hauptschneidkante 2 (13) auf gleiche Weise punktsymmetrisch ausgebildet sind,
wobei die Hauptschneidkante 1 (12) ausgehend von einem zylinderförmigen Teil des Außenumfangs (OP) ausgebildet ist, und
wobei die Hauptschneidkante 2 (13) ausgehend von den Außenumfangsrillen (G1, G2) ausgebildet ist und so ausgebildet ist, dass sie kürzer ist als die Hauptschneidkante 1 (12).

4. Hohlbohreinsatz (11) für medizinische Verwendung nach einem der Ansprüche 1 bis 3, wobei die beiden Nebenschneidkanten (14, 15) aus einer Nebenschneidkante 1 (14) und einer Nebenschneidkante 2 (15) bestehen,
wobei die Nebenschneidkante 1 (14) und die Nebenschneidkante 2 (15) um 90° oder mehr hinter der Hauptschneidkante 1 (12) und der Hauptschneidkante 2 (13) relativ zur Drehrichtung des Hohlbohreinsatzes (11) positioniert sind, und
wobei die Nebenschneidkante 1 (14) und die Nebenschneidkante 2 (15) auf einer gleichen geraden Linie in radialer Richtung im zylinderförmigen Teil ausgebildet sind.

5. Hohlbohreinsatz (11) für medizinische Verwendung nach einem der Ansprüche 1 bis 4, wobei ein zum Zentrum hin geneigter Winkel α1 der Hauptschneidkanten (12, 13) und ein zum Zentrum hin geneigter Winkel α2 der Nebenschneidkanten (14, 15) die Beziehung 0°<α1<α2 erfüllen.

6. Hohlbohreinsatz (11) für medizinische Verwendung nach einem der Ansprüche 1 bis 5, wobei ein Neigungswinkel (Θ) zwischen der Hauptschneidkante 1 (12) und der Hauptschneidkante 2 (13) in einer Richtung ausgebildet ist, in der Späne aufgrund der Drehung des Hohlbohreinsatzes (11) zu einer Seite des Außenumfangs (OP) abgeführt werden, und als Ergebnis die beim Bohrprozess der Hauptschneidkante 1 (12) erzeugten Späne von einer Seite einer Spanfläche der Hauptschneidkante 1 (12) über Außenumfangsrillen (G3) in Späneabführrillen 1 (G1) abgeführt werden, wobei die Späneabführrillen (G1, G2) Späneabführrillen 1 (G1), die an der Seite der Spanflächen der Nebenschneidkanten (14, 15) angeordnet sind, und Späneabführrillen 2 (G2) umfassen, die an den Seitenflächen an einer Seite der Hinterflächen angeordnet sind,
wobei die Späneabführrillen 1 (G1) sanft in Richtung von der Seite der Hinterflächen der Hauptschneidkanten (12, 13) am Spitzenende (TE) des Hohlbohreinsatzes (11) zum distalen Ende (DE) des Hohlbohreinsatzes (11) verlaufen, und mit den spiralförmigen Außenumfangsrillen (G1, G2) verbunden sind, die sich zur Seite der Spanflächen der Hauptschneidkanten (12, 13) hin mit einer Verdrehung zur Seite des Schafts fortsetzen, der sich am distalen Ende (DE) des Bohreinsatzes (11) befindet, und
wobei die Späneabführrillen 2 (G2) dieselbe Windungsrichtung und denselben Windungswinkel wie die Späneabführrillen 1 (G1) aufweisen.

7. Verbundbohreinsatz, umfassend einen Hohlbohreinsatz (11) nach einem der Ansprüche 1 bis 6 und Bohreinsatz mit kleinem Durchmesser, der in den Hohlteil (H) des Hohlbohreinsatzes (11) eingesetzt ist.

8. Hohlbohreinsatz (11) für medizinische Verwendung nach Anspruch 1, wobei sich Rillen, die äußere Umfangsschneidkanten (G1A) mit einer Tiefe bilden, die einer Tiefe der Außenumfangsrillen (G3) entspricht oder
geringer ist als diese, in verdrehter Weise in Richtung von dem Spitzenende (TE) zum distalen Ende (DE) des Hohlbohreinsatzes (11) erstrecken,
wobei die Rillen, die die äußeren Umfangsschneidkanten (G1B) bilden, die vier Schneidkanten (12, 13, 14, 15) und die Außenumfangsrillen (G3) durchqueren, und
wobei die Spiralrichtung der Außenumfangsrillen (G3) dieselbe ist wie die Verdrehungsrichtung der Rillen, die die äußeren Umfangsschneidkanten (G1A) bilden.

9. Hohlbohreinsatz (11) für medizinische Verwendung nach Anspruch 8, wobei zwei Schneidkanten (112, 113) unter den vier Schneidkanten (112, 113, 114, 115), die in einem Abstand vom Zentrum ausgebildet sind, Hauptschneidkanten (112, 113) sind, und die anderen beiden Schneidkanten (114, 115), die um 90° oder mehr hinter den Hauptschneidkanten (112, 113) auf einer durch das Zentrum verlaufenden geraden Linie ausgebildet sind, Nebenschneidkanten (114, 115) sind,
wobei ein Winkel α1 der zum Zentrum geneigten Hauptschneidkanten (112, 113) und ein Winkel α2 der zum Zentrum geneigten Nebenschneidkanten (114, 115) die Beziehung α1=α2=0 erfüllen, wobei die Nebenschneidkanten (114, 115) mit Spantaschen versehen sind, und
wobei eine Spiralrichtung der Außenumfangsrillen (G3) in positiver Richtung verläuft.

10. Hohlbohreinsatz (11) für medizinische Verwendung nach Anspruch 8 oder 9, wobei die beiden Hauptschneidkanten (112, 113) aus einer Hauptschneidkante 1 (112) und einer Hauptschneidkante 2 (113) bestehen,
wobei die Hauptschneidkante 1 (112) und die Hauptschneidkante 2 (113) auf gleiche Weise punktsymmetrisch ausgebildet sind,
wobei die Hauptschneidkante 1 (112) ausgehend von einem zylinderförmigen Teil des Außenumfangs (OP) ausgebildet ist, und
wobei die Hauptschneidkante 2 (113) ausgehend von den Außenumfangsrillen (G3) ausgebildet ist und so ausgebildet ist, dass sie kürzer ist als die Hauptschneidkante 1 (112).

11. Hohlbohreinsatz (11) für medizinische Verwendung nach einem der Ansprüche 8 bis 10, wobei die beiden Nebenschneidkanten (114, 115) aus einer Nebenschneidkante 1 (114) und einer Nebenschneidkante 2 (115) bestehen,
wobei die Nebenschneidkante 1 (114) und die Nebenschneidkante 2 (115) um 90° oder mehr hinter der Hauptschneidkante 1 (112) und der Hauptschneidkante 2 (113) relativ zur Drehrichtung des Hohlbohreinsatzes (11) positioniert sind, und
wobei die Nebenschneidkante 1 (114) und die Nebenschneidkante 2 (115) auf einer gleichen geraden Linie in radialer Richtung im zylinderförmigen Teil ausgebildet sind.

12. Verbundbohreinsatz, umfassend einen Hohlbohreinsatz (11) nach einem der Ansprüche 8 bis 11 und einen Bohreinsatz mit kleinem Durchmesser, der in den Hohlteil (H) des Hohlbohreinsatzes (11) eingesetzt ist.

## Revendications

1. Foret creux (11) à usage médical comprenant
une partie creuse (H) qui pénètre dans un centre axial dans une direction axiale depuis une extrémité de pointe (TE) jusqu'à une extrémité distale (DE) ; et
quatre arêtes de coupe (12, 13, 14, 15) au niveau d'une section de l'extrémité de pointe (TE),
dans lequel des rainures périphériques externes (G1, G2, G3) en forme d'arc sont formées de manière hélicoïdale dans une périphérie externe (OP) de forme cylindrique,
dans lequel une direction hélicoïdale des rainures périphériques externes (G1, G2, G3) est une direction vers l'avant, dans lequel les quatre arêtes de coupe (12, 13, 14, 15) ont une forme linéaire inclinée depuis la périphérie externe (OP) vers un centre du foret creux,
dans lequel deux arêtes de coupe (12, 13) parmi les quatre arêtes de coupe qui sont formées à une distance du centre sont des arêtes de coupe principales,
dans lequel deux autres arêtes de coupe (14, 15) qui sont formées à 90 ° ou plus en arrière des arêtes de coupe principales (12, 13) sur une ligne droite à travers le centre sont des arêtes de coupe secondaires (14, 15), dans lequel les arêtes de coupe secondaires (14, 15) sont pourvues de poches à dépouille (GP).

2. Foret creux (11) à usage médical selon la revendication 1, dans lequel des rainures d'évacuation de copeaux (G1, G2) pour évacuer des copeaux générés par les arêtes de coupe (12, 13, 14, 15) pendant l'utilisation du foret creux (11) s'étendent de manière torsadée dans une direction allant de l'extrémité de pointe (TE) à l'extrémité distale (DE) du foret creux (11),
dans lequel les rainures d'évacuation de copeaux traversent les quatre arêtes de coupe et les rainures périphériques externes, et
dans lequel la direction hélicoïdale des rainures périphériques externes est identique à une direction de torsion des rainures d'évacuation de copeaux.

3. Foret creux (11) à usage médical selon la revendication 1 ou 2, dans lequel les deux arêtes de coupe principales (12, 13) sont constituées d'une arête de coupe principale 1 (12) et d'une arête de coupe principale 2 (13), dans lequel l'arête de coupe principale 1 (12) et l'arête de coupe principale 2 (13) sont formées de manière symétrique par rapport à un point, d'une manière identique,
dans lequel l'arête de coupe principale 1 (12) est formée à partir d'une partie en forme de cylindre de la périphérie externe (OP), et
dans lequel l'arête de coupe principale 2 (13) est formée à partir des rainures périphériques externes (G1, G2) et est formée pour être plus courte que l'arête de coupe principale 1 (12).

4. Foret creux (11) à usage médical selon l'une quelconque des revendications 1 à 3, dans lequel les deux arêtes de coupe secondaires (14, 15) sont constituées d'une arête de coupe secondaire 1 (14) et d'une arête de coupe secondaire 2 (15),
dans lequel l'arête de coupe secondaire 1 (14) et l'arête de coupe secondaire 2 (15) sont positionnées à 90 ° ou plus en arrière de l'arête de coupe principale 1 (12) et de l'arête de coupe principale 2 (13) par rapport à une direction de rotation du foret creux (11), et
dans lequel l'arête de coupe secondaire 1 (14) et l'arête de coupe secondaire 2 (15) sont formées sur une ligne droite identique dans une direction radiale dans la partie en forme de cylindre.

5. Foret creux (11) à usage médical selon l'une quelconque des revendications 1 à 4, dans lequel un angle α1 des arêtes de coupe principales (12, 13) incliné vers le centre et un angle α2 des arêtes de coupe secondaires (14, 15) incliné vers le centre satisfont à la relation 0°<α1<α2.

6. Foret creux (11) à usage médical selon l'une quelconque des revendications 1 à 5, dans lequel un angle incliné (Θ) formé par l'arête de coupe principale 1 (12) et l'arête de coupe principale 2 (13) est formé dans une direction dans laquelle des copeaux sont évacués vers un côté de la périphérie extérieure (OP) en raison de la rotation du foret creux (11), et en conséquence, des copeaux générés par un processus de perçage de l'arête de coupe principale 1 (12) sont évacués depuis un côté d'un angle de coupe de l'arête de coupe principale 1 (12) vers des rainures d'évacuation de copeaux 1 (G1) par l'intermédiaire de rainures périphériques externes (G3),
dans lequel les rainures d'évacuation de copeaux (G1, G2) comprennent des rainures d'évacuation de copeaux 1 (G1) prévues au niveau du côté des arêtes de coupe secondaires (14, 15) et des rainures d'évacuation de copeaux 2 (G2) prévues sur les surfaces latérales au niveau d'un côté de talons,
dans lequel les rainures d'évacuation de copeaux 1 (G1) sont reliées en douceur dans la direction allant du côté des talons des arêtes de coupe principales (12, 13) au niveau de l'extrémité de pointe (TE) du foret creux (11) jusqu'à l'extrémité distale (DE) du foret creux (11), et sont reliées aux rainures périphériques extérieures (G1, G2) hélicoïdales se prolongeant jusqu'au côté des arêtes de coupe principales (12, 13) avec une torsion vers le côté de la tige, qui est au niveau de l'extrémité distale (DE) du foret (11), et
dans lequel les rainures d'évacuation de copeaux 2 (G2) ont une direction d'hélice identique et un angle d'hélice identique à ceux des rainures d'évacuation de copeaux 1 (G1).

7. Foret composite comprenant un foret creux (11) selon l'une quelconque des revendications 1 à 6 et un foret de petit diamètre inséré dans la partie creuse (H) dudit foret creux (11).

8. Foret creux (11) à usage médical selon la revendication 1, dans lequel des rainures formant des arêtes de coupe périphériques externes (G1A) avec une profondeur équivalente ou inférieure à une profondeur des rainures périphériques externes (G3) s'étendent de manière torsadée dans la direction allant de l'extrémité de pointe (TE) à l'extrémité distale (DE) du foret creux (11),
dans lequel les rainures formant des arêtes de coupe périphériques externes (G1B) sont transversales aux quatre arêtes de coupe (12, 13, 14, 15) et aux rainures périphériques externes (G3), et
dans lequel la direction hélicoïdale des rainures périphériques externes (G3) est identique à la direction de torsion des rainures formant des arêtes de coupe périphériques externes (G1A).

9. Foret creux (11) à usage médical selon la revendication 8, dans lequel deux arêtes de coupe (112, 113) parmi les quatre arêtes de coupe (112, 113, 114, 115) qui sont formées à une distance du centre sont des arêtes de coupe principales (112, 113), et deux autres arêtes de coupe (114, 115) qui sont formées à 90 ° ou plus en arrière des arêtes de coupe principales (112, 113) sur une ligne droite à travers le centre sont des arêtes de coupe secondaires (114, 115),
dans lequel un angle α1 des arêtes de coupe principales (112, 113) incliné vers le centre et un angle α2 des arêtes de coupe secondaires (114, 115) incliné vers le centre satisfont à la relation α1=α2=0, dans lequel les arêtes de coupe secondaires (114, 115) sont pourvues de poches à dépouille, et
dans lequel la direction hélicoïdale des rainures périphériques externes (G3) est une direction positive.

10. Foret creux (11) à usage médical selon la revendication 8 ou 9, dans lequel les deux arêtes de coupe principales (112, 113) sont constituées d'une arête de coupe principale 1 (112) et d'une arête de coupe principale 2 (113),
dans lequel l'arête de coupe principale 1 (112) et l'arête de coupe principale 2 (113) sont formées de manière symétrique par rapport à un point, d'une manière identique,
dans lequel l'arête de coupe principale 1 (112) est formée à partir d'une partie en forme de cylindre de la périphérie externe (OP), et
dans lequel l'arête de coupe principale 2 (113) est formée à partir des rainures périphériques externes (G3) et est formée pour être plus courte que l'arête de coupe principale 1 (112).

11. Foret creux (11) à usage médical selon l'une quelconque des revendications 8 à 10, dans lequel les deux arêtes de coupe secondaires (114, 115) sont constituées d'une arête de coupe secondaire 1 (114) et d'une arête de coupe secondaire 2 (115),
dans lequel l'arête de coupe secondaire 1 (114) et l'arête de coupe secondaire 2 (115) sont positionnées à 90 ° ou plus en arrière de l'arête de coupe principale 1 (112) et de l'arête de coupe principale 2 (113) par rapport à une direction de rotation du foret creux (11), et
dans lequel l'arête de coupe secondaire 1 (114) et l'arête de coupe secondaire 2 (115) sont formées sur une ligne droite identique dans une direction radiale dans la partie en forme de cylindre.

12. Foret composite comprenant un foret creux (11) selon l'une quelconque des revendications 8 à 11 et un foret de petit diamètre inséré dans la partie creuse (H) dudit foret creux (11).
